# EUROPEAN PATENT SPECIFICATION

(11) **EP 3 975 871 B1**
(45) Date of publication and mention of the grant of the patent: **24.06.2026**
(21) Application number: 20729041.2
(22) Date of filing: 26.05.2020
(51) Int. Cl.: A61B 10/00, A61B 5/00

(54) **BODY TEMPERATURE DIAGNOSTICS**
KÖRPERTEMPERATURAUSWERTUNG
DIAGNOSTIQUE DE LA TEMPERATURE D'UN CORP

(30) Priority: 26.05.2019 DE 102019114044
(43) Date of publication of application: 06.04.2022
(73) Proprietor: VivoSensMedical GmbH, 04229 Leipzig (DE)
(72) Inventor: ALEXANDER, Henry, 04299 Leipzig (DE); ZIEGNER, Andreas, 04229 Leipzig (DE)
(74) Representative: Schreiter, Christoph
(86) International application number: PCT/EP2020/064613
(87) International publication number: WO 2020/239789

(56) References cited:
- WO-A1-2016/183311
- DE-A1- 19 524 966
- US-A1- 2016 270 768
- US-A1- 2017 235 443

## Description

### Field of the invention

The present disclosure relates to a method and a system for monitoring of the menstrual cycle of a female. In particular, the present disclosure relates to a method and a system for determining the hormone status and the fertility status of a female based on body temperatures.

### Introduction

Fertility is one of the most important health care aspects in the society. Fertility and fecundity are associated with several factors such as psychological, environmental and chemical factors. The increasing influence of environmental factors and knowledge about the associated risks to such factors are leading to a growing need for methods by which the fertility of the population can be assessed without significant encroachment of the personal circumstances of test persons. Measurement methods systems for the evaluation of the menstrual cycle and fertility of women are much needed that will not encroach on their daily routines.

Determining fertility either for family planning or for contraception is a critical approach of the female health care system and precise determination of ovulation is required. The menstrual cycle is a complex interplay of many hormones and other body functions. So far, some indirect methods to determine the ovulation in women are based on serum hormone levels or measurement of the basal body temperature. The method using serum hormone levels is reliable but the employment of laboratory diagnostic methods that are required for these measurements in urine leads to substantial costs.

Otherwise, a postovulatory rise in the temperature of about 0.5°C (+/- 0.1 °C) has been documented in the evaluation of the course of the menstrual cycle. This temperature rise is due to circadian variations of the body core temperature of about ± 0.5 ºC. Conventional methods aim to identify and determine this variation in body core temperature. These conventional methods are based on the estimation of a basal temperature, the lowest temperature during a day. Due to many other influences on the body temperature, the basal temperature is mostly measured in the morning after wake-up and involves an inconvenient temperature measurement right after waking up. The temperature measurement must then be repeated every day. A change in this basal temperature is used as an indication for ovulation. These methods are not very reliable.

US 2016/0331244 A1 describes a system and method for determining a body core temperature including multiple sensors in combination with analytic technologies. Temperature amplitudes are used to determine basal body temperature changes.

US 2016/270768 A1 from the applicant describes methods for analysing specific features in temperature data curves based on body core temperatures.

In WO2016/183311 A1, body temperature is used to determine a fertility parameter by analysing the temperature amplitudes.

There is a need to provide a more reliable determination of the menstrual cycle of a female and to determine the actual fertility status of the woman or female.

### Summary of the invention

The present disclosure suggests a method and a system for determining the status of a biomarker in a mammal or human being as outlined in the independent claims. Optional additional features are presented in the dependent claims.

In one aspect, the method comprises the steps of claim 1.

In some examples, the fertility states may relate to at least one of menstrual cycle diagnostics, CFG score, contraception and pregnancy or any combination thereof.

In another aspect, the system comprises a temperature measurement device for determining and recording a series of temperature data points relating to a body temperature of the female and an analysing tool for analysing the series of temperature date points, wherein the series of temperature data points is transferred from the temperature measurement device to the analysing tool. The analysing tool performs at least the method steps of claim 1.

### Description of the figures

The invention may be better understood when reading the detailed description of examples of the present disclosure which is given with respect to the accompanying figures in which:
Figs. 1 shows a pessary that may be used with the present disclosure
Figs. 2a and 2b show examples of temperature curves that may be determined with the analysing system of Fig. 1;
Fig. 3 shows an example of how basal temperatures can be derived from a temperature curve;
Figure 4a and 4b show how the temperature amplitude may be determined from a set of temperature data points;
Figure 5a and 5b show how the temperature amplitude may be determined with the correction for absolute temperature values;
Fig 6a to 6e shows different examples for different variations in hormone levels;.
Fig. 7 shows an example of temperature curve and temperature amplitudes indicating a commencing pregnancy; Fig 8 shows how different variations in the hormone level may be classified;
Fig. 9 shows an example of how the temperature amplitude is related to the active progesterone; and
Figs. 10 and 11 show examples how temperature amplitude variations may be correlated to other parameters, such as pH-values and nadir values.

### Detailed description

Examples of the present disclosure will now be described in more detail. It is to be understood that the described examples and the examples shown in the figures are purely illustrative and a person skilled in the art will amend the examples according to specific requirements. It is not necessary to implement all features shown in the examples and a person skilled in the art will combine features shown or described with respect to one figure with examples shown in other figures or described elsewhere in the present disclosure.

The present disclosure relates in one example to a method and a system for determining a status of a biomarker in a mammal or a human being. In one aspect, the biomarker is a progesterone response of the mammal or human being, in particular the progesterone response of a woman. The progesterone response correlates to active progesterone, that is progesterone that has caused a response within the body, for example if the progesterone binds to a receptor and triggers further body reactions. Free progesterone, in contrast, is the amount of progesterone available in blood.

The method and system performing the method comprises determining, for a plurality of first time intervals, a continuous series of temperature data points relating to a body temperature.

The method comprises further determining, in the series of data points, for the plurality of first time intervals, differences between a temperature maximum and a temperature minimum to obtain a plurality of temperature amplitudes, and determining a variation in the temperature amplitude, wherein the variation in the temperature amplitude indicates a change in the status of the biomarker. A temperature amplitude may be determined for each one of the plurality of time intervals. This may include to leave out some of the first time interval if data are not correct. The term each one of the first time intervals may be understood by an amplitude value is determined or calculated for those of the plurality of time intervals, where such a determination is reasonable.

The method comprises determining a series of representative temperature values for the plurality of first time intervals and subtracting the series of representative temperature values from the continuous series of temperature data points relating to the body temperature to obtain a series of temperature fluctuations. A difference between a temperature maximum and a temperature minimum is determined in the series of temperature fluctuations for the plurality of first time intervals to obtain a plurality of temperature amplitudes.

Determining a variation in the temperature amplitude comprises determining if a first portion of the series of temperature amplitudes varies from a second portion of the series of temperature amplitudes indicating a change in the status of the biomarker. It may be advantageous and increase reliability if a first portion and second portion is determined. An average or mean value may be determined for the first portion and for the second portion, respectively. The first portion of the plurality of temperature amplitudes may be temperature amplitudes determined in subsequent first time intervals. Similar, the second portion of the plurality of temperature amplitudes may be temperature amplitudes determined in subsequent first time intervals. The first portion and second portion may comprise different number of temperature amplitudes and may relate to different length in time. Measurement of temperature amplitudes that have faulty or erroneous values may be filtered or otherwise excluded from the first portion or the second portion.

Determination of the body temperature may comprise measuring the temperature and storing or recording the temperature or data representing or relating to the temperature.

The body temperature may be a body core temperature that is advantageously measured in a body orifice. The continuous series of temperature data points relate to the body core temperature. For example, the body core temperature measured in the vaginal channel of a female and the continuous series of temperature data points relate to the body core temperature. A pessary with temperature sensor may be used as an example. Body core temperatures measured inside a body orifice are more reliable compared to measurement on the skin. Temperature measurements on the skin are influenced by external parameters such as the positioning on the skin, external temperatures, external heat and light sources as well as body reactions such as transpiration, sweating or body movements. Skin temperature measurements usually give only approximate measurements and are not very reliable.

Determining the continuous series of temperature data points comprises measuring a temperature data in intervals of every 15 minutes or less continuously over at least 10 days. The series of temperature data points may be determined continuously, for example over several days, a week or the entire menstrual cycle. A typical time interval may be a measurement every 5 minutes resulting in about 288 temperature data points per day (within 24 hours). The time interval may be greater or less than five minutes, for example every minute or every 10 or 15 minutes resulting in more or less temperature data points per day. Temperature variations in shorter time intervals do not give additional information and measurement in time intervals as mentioned above are considered to result in a continuous temperature data curve. It is advantageous, however, to monitor the temperature continuously over the whole day, during night and day. In case of a female ovulatory cycle the temperature may be measured over the entire menstrual cycle of the female. A continuous temperature data curve can be evaluated from these temperature data points representing the actual body core temperature of the female.

The continuous series of temperature data points may be determined for a plurality of first time intervals. The first time intervals may follow directly one after another without any interruption such as a 24hrs. day and one time interval may correspond to 24hrs. or a day. The first time intervals may be longer or shorter than a day. A daily measurement, however has bee found useful for every day life of the user. For example, the first time intervals may be started by the insertion of the measurement devices and may be ended by removing the measurement device from the body for readout about 24 one day later. If stated herein that a value is determined for the first time intervals or for each one of the first time intervals, this may be understood as a value is determined for a respective time interval of the first time intervals. The term each one may include that some of the time intervals may not result in values and may be excluded from the series.

The difference between the temperature maximum and the temperature minimum may be termed daily temperature amplitude. In other examples, the time intervals may be interrupted for example by removing the measurement device from the body for read-out. In some application only day or only night periods may be used as first time intervals. A continuous measurement without major interruption will, however, deliver more reliable data.

A series of representative temperature values is determined for the plurality of first time intervals. The series of representative values can be a series average temperature values, of Nadir values or if another value representing the absolute temperature for example in each one of the first time intervals. The series of representative values is subtracted from the continuous series of temperature data points relating to the body temperature to obtain a series of temperature fluctuations. By substracting the representative temperature values, effects from temperature variation can be eliminated. It is an advantage to measure core body temperatures in a body orifice to obtain more precise temperature variations.

In the series of temperature fluctuations, for each one of one of the plurality of first time intervals, a difference between a temperature maximum and a temperature minimum is determined to obtain a plurality of temperature amplitudes. The temperature amplitudes are a relative value that is independent of the absolute temperature and only monitors the temperature change or difference over the first time interval. This indicates the temperature variation of the first time interval, for example the temperature variation within a day or 24 hrs. Influences of rising temperature or of decreasing body temperatures and in some cases of artifacts can be excluded if the series of temperature fluctuations is used for the determination of the series of amplitudes.

A series of temperature amplitudes from the plurality of temperature amplitudes. The method may comprise identifying a significant change in the representative temperature value and correlating the significant the first portion of the series of temperature amplitude and the second portion of the series of temperature amplitude. One temperature amplitude value may be representative for each first time interval. If the first time interval is one day, the temperature amplitude may represent the amplitude or temperature variation of that day and may be termed daily temperature amplitude.

A variation in the temperature amplitude is then determined. A series or subset of temperature amplitude is determined from the plurality of temperature amplitudes and a variation in the temperature amplitude over time may be identified. A variation of a first portion of the series of temperature amplitudes from a second portion of the series of temperature amplitudes indicates a change in the status of the biomarker. The first portion and the second portion may be represented by a first mean or average amplitude and a second mean or average temperature amplitude of the temperature amplitudes, respectively. The variation between the first portion of the series of temperature amplitudes and the second portion of the series of temperature amplitude may be at least one of a variation in the average amplitude of the temperature amplitudes, a variation in the change rate of the temperature amplitudes, an increase the temperature amplitudes or a decrease in the temperature amplitudes.

The hormone biomarker is progesterone in the examples shown and relating to monitoring a female menstrual cycle. The disclosure, however, may also relate to the determination of other hormones or other biomarkers that can be determined by a change in the temperature amplitude. A first average amplitude of the first portion may be larger than a second average amplitude of the second portion indicating a higher active progesterone level in the second portion.

The female menstrual cycle is determined by complex endocrinological systems. The current method to measure a hormone status and/or a variation of hormone levels are one point measurements, which only provide an edocrinological spot point of the very moment when the measurement was taken. These hormone levels undergo many variations and a spot view therefore is insufficient and unprecise. A detailed and individual diagnostic of the female cycle is only very limited.

The female cycle is a seismograph for women's health. Therefore an individual and continuous method to scan the female is needed to precise diagnose beside others, fertility and infertility, hormone disorders and insufficiencies, Polycystic ovary syndrome (PCOS).

The present disclosure seeks to provide individual diagnostic information about the active progesterone hormone status of the female cycle to provide a diagnosis and a prognose on beside others, fertility and infertility, hormone disorders and insufficiencies, PCOS.

As an example, determining fertility either for family planning or for contraception is a critical approach of the female health care system and precise determination of ovulation and/or the current status of the menstrual cycle is required. The present disclosure seeks to provide diagnostic information about the status of the menstrual cycle and a prognosis of the "fertility window".

In addition to ovulation, a complex interplay of the endocrinal system is required to establish pregnancy in which hormones and the variation of hormone levels at the right time play an important role.

The present disclosure relates in one example to a method and a system for determining a progesterone level, in particular a level of active progesterone and the progesterone response in a female by measuring a body core temperature. The method comprises determining, for a plurality of first time intervals, a continuous series of temperature data points relating to a body temperature, determining, in the series of data points, for each one of one of the plurality of first time intervals, a differences between a temperature maximum and a temperature minimum to obtain a plurality of temperature amplitudes, and determining if a first average temperature amplitude in a first subset or first portion of the plurality of temperature amplitudes differs from a second average temperature amplitude in a second subset or second portion of the plurality of temperature amplitudes, a difference indicating a change in the progesterone response.

The inventors found the temperature amplitude can be used as a measure for the progesterone response or the active progesterone in the body. The active progesterone or progesterone response can be different to the absolute progesterone level, as for example measured in blood. The absolute progesterone level in blood may further increase while all progesterone receptors are activated and the progesterone response is saturated. While the absolute progesterone level indicates how much progesterone is available in the body. The progesterone response is a measure for the progesterone that actually triggered body reactions or was taken up by progesterone receptors.

The body core temperature may be measured by a temperature sensor placed in the vaginal channel of the female. The body core temperature may also be measured in other body orifices and may not be restricted to females. The body core temperature is the most reliable temperature measurement of a human or animal and is by far more precise than temperature measurements on other places, for example on the skin. Device carried on the wrist or sensor plasters devices are often influenced by external or environmental conditions and are less reliable.

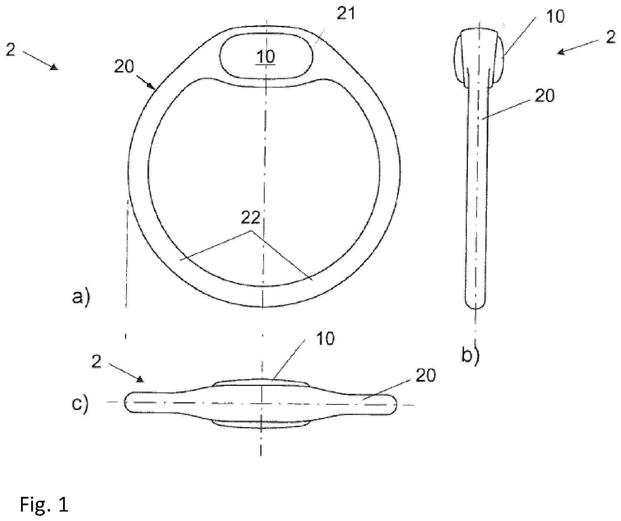

An example of a temperature sensor for measuring the body core temperature that may be used with the present disclosure is shown in Figs 1a to 1c and described in US 2013/0237771 (EP 2567680). The temperature sensor 10 may have the form of a pessary or may be attached to a pessary 20 placed in the vaginal channel of the female. The temperature sensor 10 can measure the actual body core temperature of the user inside the vaginal channel. The temperature sensor is able to measure and record circamensual (30 days and more) a series of body core temperature data points. The temperature sensor attached to the pessary provides high comfort for the user and high data reliability and is therefore advantageous for the method and system of the present disclosure. The method and system of the present disclosure, however, may be used with other body temperature sensors that measure continuous series of body core temperature data points inside the body or series of temperature values at other places of the body even though the temperature measurement in other places may be less precise.

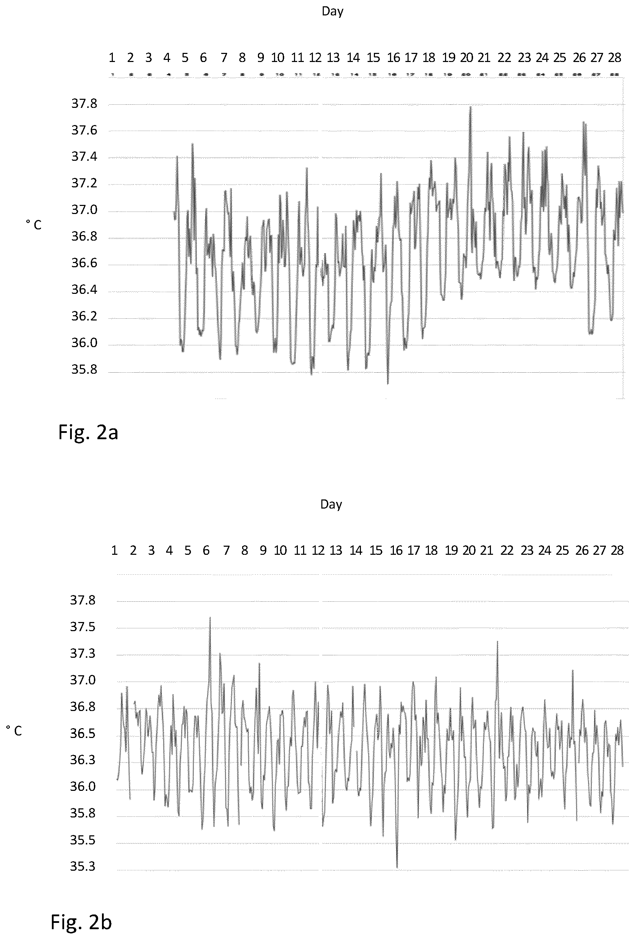

It is advantageous to monitor the temperature continuously over the whole day, during night and day, and if possible over the entire menstrual cycle of the female. A continuous temperature data curve can be evaluated from these temperature data points representing the actual body core temperature of the female. Examples for continuous temperature data curves are shown in Figures 2a and 2b, where 288 temperature data points per day were measured and recorded continuously every 5 minutes over a menstrual cycle (about 30 days or more).

Conventional approaches, for example, measure the temperature data only during limited time intervals for example after wake-up or during night sleep phases. The temperature sensor is removed for read out in the morning. These approaches try to estimate a basal temperature based on the lowest temperatures measured during the sleep phase. These methods aim to determine a basal temperature which is often not well defined. This method is not very reliable. In addition, these methods are also influenced by the behaviour and the conditions of the user. Not every night and sleep phase is the same and variations in the life style, the amount and time of sleep may have a strong influence on the measured values. These conventional approaches usually take no measurements during day time or disregard any measurements taken during day time as day time activities usually increase the body temperature and are therefore not useful in the determination of the basal temperature.

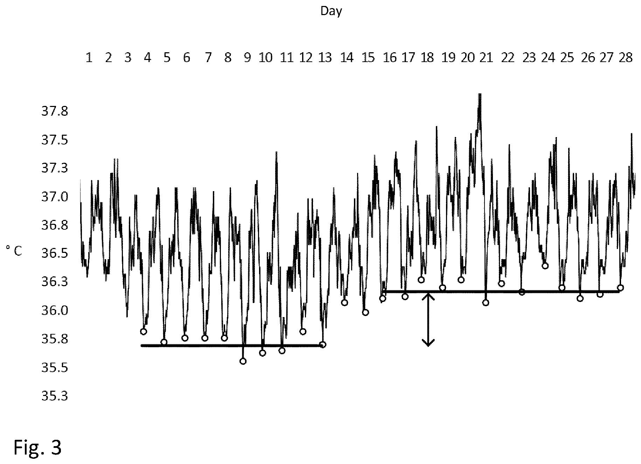

Basal temperature may also be determined from continuous series of data points as indicated in Fig. 3 with the present disclosure. The lowest data points, i.e. minima in the series of data points are determined for every day in the menstrual cycle. A raise or drop of these basal temperature values may be determined. These basal temperature changes are a well-known and widely used indicator relating to ovulation. The natural variation of the basal temperature limits the reliability of this method. The basal temperature, however, may be used as an additional indicator in combination with other indicators and features in the series of data points as described below.

The inventors found that the temperature curve contains additional information beyond the basal temperature and specific features in the temperature data curve can be analysed. Examples of a feature analysis in a series of temperature data points are described in WO 2015/044398 A1.

The present disclosure suggests the use of a continuous series of temperature data points of the body core temperature measured in the vaginal channel of the female which are determined and recorded continuously over a plurality of days, over weeks or circamensual to determine temperature curves of the body core temperature over long periods. Depending on the application, the sensor may remain in the vagina for the entire menstrual cycle and the recorded data may be read out at the end of the menstrual cycle. Alternatively, the recorded temperature data may be read out from time to time, for example once a week, once a day or other time intervals. The measurement and read-out of the actual temperature data enables a "real-time" determination of the status of the menstrual cycle.

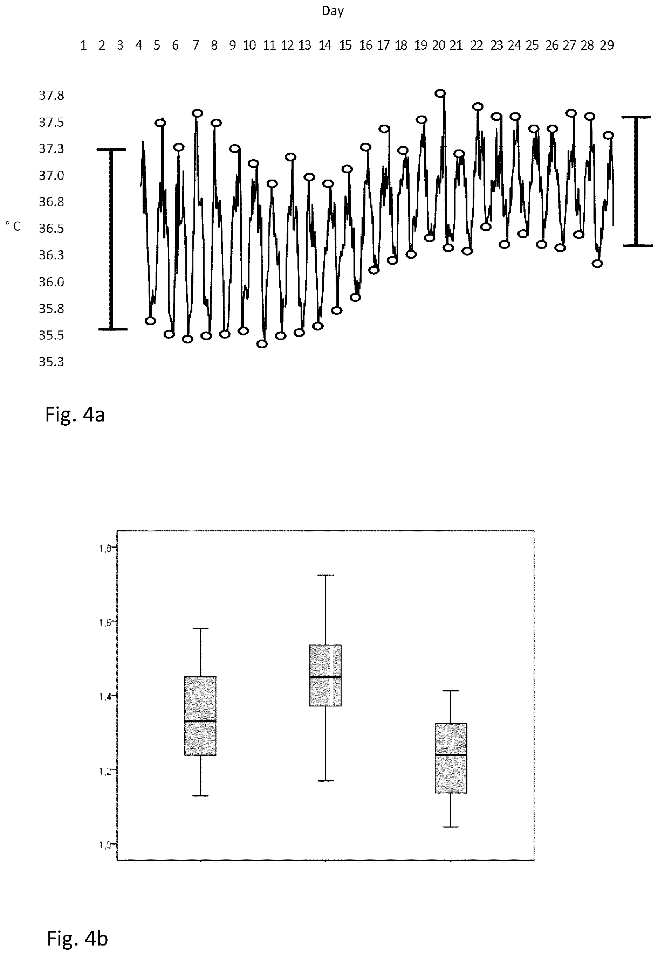

These recorded temperature curves are analysed and/or evaluated. According to one aspect of the present disclosure, the continuous series of temperature data points is analysed for temperature amplitudes. A temperature amplitude is the temperature difference between a maximum temperature value and a minimum temperature value within a pre-defined time interval. The pre-defined time interval may be typically a day or 24 hours and the corresponding temperature amplitude may be termed daily temperature amplitude. The (daily) temperature amplitude is independent of the absolute temperature value and thus independent of effects influencing the body temperature and the basal temperature. Figure 4a shows how the temperature amplitude may be determined from a set of temperature data points.

Determining and recording, for a plurality of first time intervals, a continuous series of temperature data points relating to a body temperature may comprise determining representative temperature values from a set of raw data relating to the temperature measured by a temperature sensor. Representative temperature values may be obtained by filtering and/or fitting these temperature raw data. For example, filtering may comprise at least one of determining a mean value, a median value from a group or a subgroup of temperature values. Filtering may also comprise low-pass filtering or a moving average filter. Fitting these temperature raw data may comprise at least one of smoothing the resulting temperature curve and approximating the temperature raw data for example by a cosinus or a sinus function. The cosinus or sinus function may have additional correction factors, if needed.

The maximum temperature value and the minimum temperature value may be determined directly from the set of temperature data points. A filter may be applied to filter out and/or delete unrealistic and faulty data points or values from the set of temperature data point. It is also possible to use more than one temperature data point but a series of temperature data points to determine the representative maximum temperature value and the representative minimum temperature value. Usual methods for data treatment may be applied if considered applicable by a person skilled in the art.

The temperature maximum and the temperature minimum may be determined based on the representative temperature values and may be representative temperature maximum and representative temperature minimum. For simplicity the term temperature maximum and temperature minimum is herein also used the representative temperature maximum and the representative temperature minimum, respectively.

The inventors found that the daily temperature amplitude and a variation of the daily temperature amplitude can be used as an indicator for the active progesterone level in particular in women, but more generally in mammals. The inventors found that the amplitude of the temperature is a measure for the response or reaction of the body to the presence of progesterone. The body response may be in some cases but may not in others relate to the presence of free progesterone in blood. Only a portion of free progesterone may bind to progesterone receptors, progesterone receptors may be saturated so that additional free progesterone can not bind to recetors or can not trigger a response. In other case triggering a signal may be interrupted or disturbed at some point in the signal cascade. In these cases free progesterone in blood may be still high but there will be no or only reduced response by the body to progesterone. The temperature amplitude is therefore a biomarker for the active progesterone level or the progesterone response and a change in the temperature amplitude indicates a change in the active progesterone . Typically, a decrease in the temperature amplitude indicates an increase in active progesterone level i.e. an increase progesterone response and an increase in the temperature amplitude in an indicator for a decrease in the active progesterone level, i.e. a decreased progesterone response.

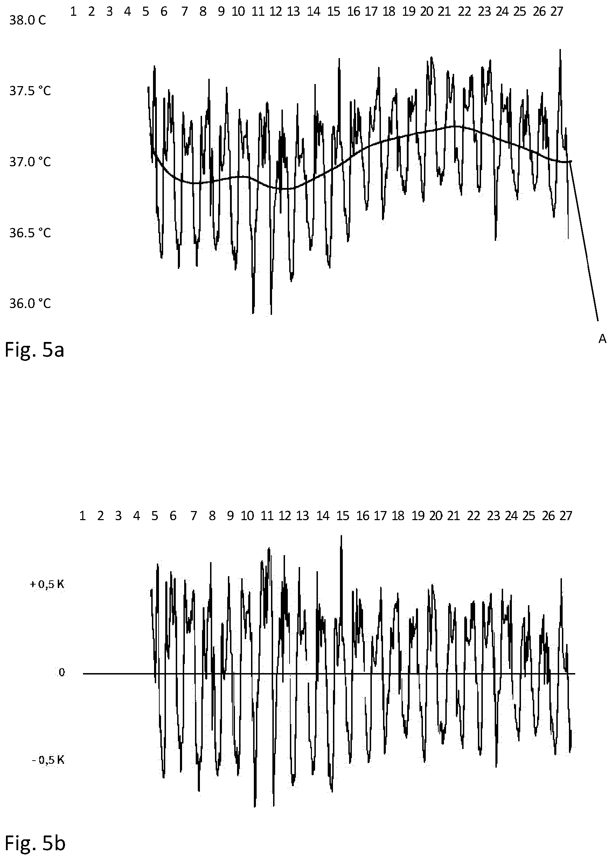

It may be advantageous to further evaluate the temperature data. Calculation of the temperature amplitude may be influenced by a variation of the body temperature, i.e. a raise or fall in the body temperature. Fig 5 shows an example of how a more precise amplitude can be obtained. This is in particular useful for a precise and/or real time measurement of the progesterone level. Fig. 5a shows a series of temperature data from one female cycle. A smoothened average or mean is calculated from these data, shown as graph A in Figure 5a. This may correspond to the average body temperature. The average body temperature may be subtracted from the series of temperature data resulting in a series of daily temperature variations or daily temperature fluctuations as shown in Figure 5b. The representative temperature maximum and representative temperature minimum and thus the daily temperature amplitude and a variation of the daily temperature amplitude may now be determined from the daily temperature variation. Using the daily temperature variation instead of the temperature data directly has the advantage that the changes in the absolute temperature occurring from health states or other influences can be excluded. The inventors found that the use of body core temperature data measured inside a body orifice yield in more precise daily temperature variations and thus in more reliable temperature amplitudes and amplitude variations.

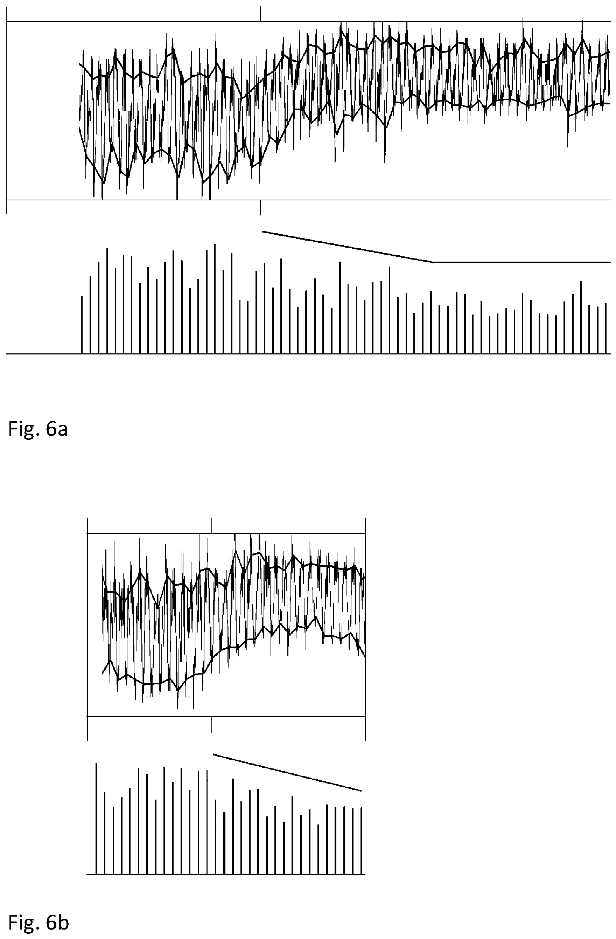

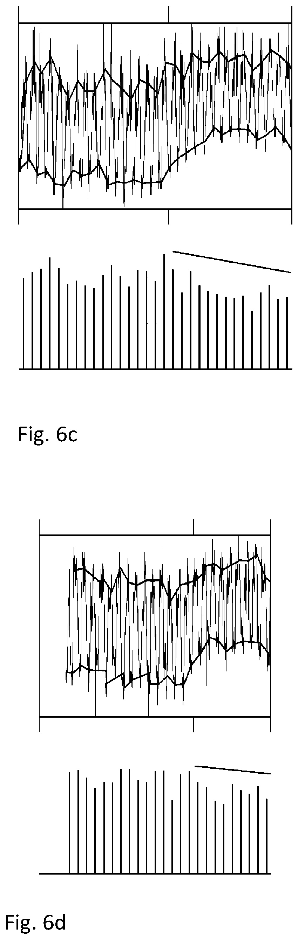

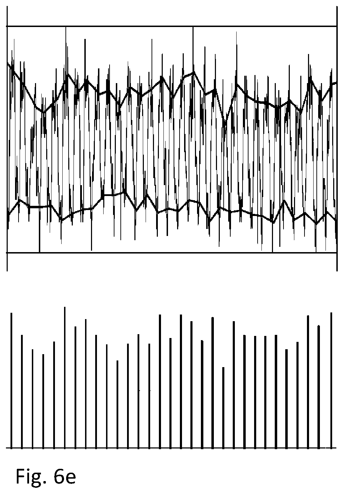

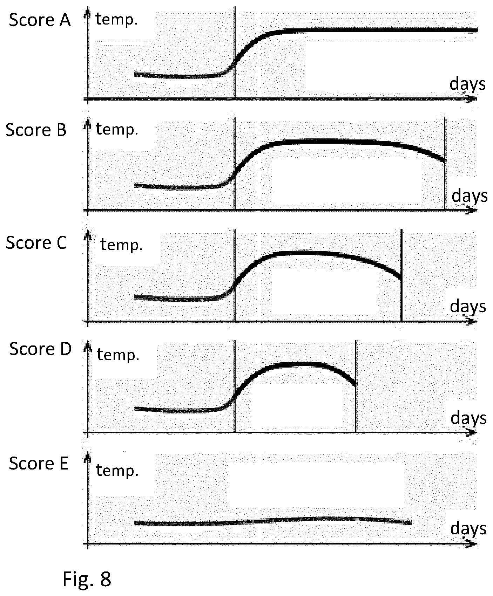

A menstrual cycle can be classified depending on the temperature and the hypothermic phase based on the progesterone level as shown in Figures 6 to 8. This method is termed Cyclofertilography^{™} or CFG-score^{™} by the applicant and classifies a menstrual cycle into different fertility classes using a fertility score. The score differentiates six classes depending on the presence and length of the luteal phase. The presence and length of the luteal phase is determined by the measurement of the progesterone level which may be derived from the temperature amplitude as described herein.

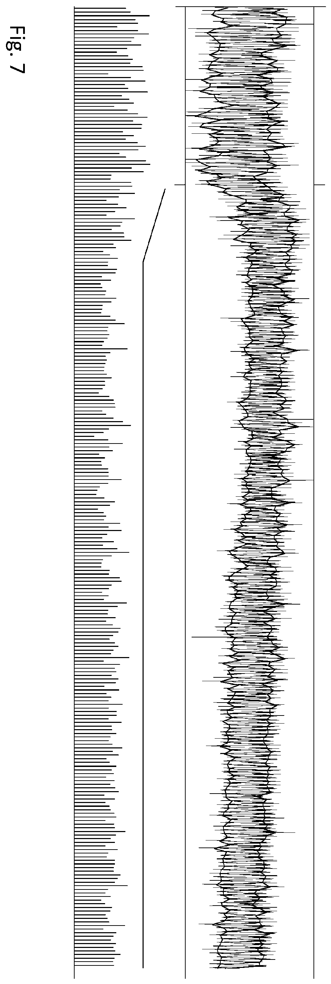

CFG-score A: If a temperature increase and/or an increase in progesterone level is determined by the reduction of the temperature amplitude and if the temperature amplitude remains low for more than 20 days, the cycle is classified as a very long hypothermic Phase, which is an indication for beginning pregnancy. The temperature amplitude may or may not remain and at a higher level during the beginning pregnancy (Fig. 6a, Fig 7). As illustrated in Fig. 7, the temperature level is slowly reduced during the beginning pregnancy while the temperature amplitude shown in the lower graph remains stable at the low level indicating that the active progesterone level remains high. Returning to Fig. 6a, the temperature level remains high while the temperature amplitude remains at a lower level. This clearly shows that the determination of the temperature amplitude can give additional information. Monitoring the reduced amplitude can help monitoring the status of pregnancy. This can help to monitor any irregular events in pregnancy during the entire course of pregnancy. An increase in daily amplitude and/or strong variation in the daily temperature may indicate issues in pregnancy that required medical attention.

CFG-score B: Fig 6b shows an example for a temperature data curve (upper graph) and a corresponding temperature amplitude (lower graph) of a fertile menstrual cycle with a long hypothermic Phase and a good possibility for a beginning pregnancy. A temperature increase is determined and the increase lasts for about 16 to 19 days with an increase in temperature for about 3-5 days and a decrease from day 16-19 on. If this category occurs frequently within one patient a disturbed early pregnancy or ovarian cyst should be excluded by advanced diagnostics..

CFG-score C: Fig 6c shows an example for a temperature data curve (upper graph) and corresponding temperature amplitude (lower graph) of a "normal" or average female menstrual cycle with a luteal phase. A temperature increase lasts for 11 to 15 days while the temperature amplitude is decreasing over the same period. This indicates a "ideal" or "normal" luteal phase. A significant decrease of the temperature amplitude over about 11 to 15 days is determined and also indicates a "normal" fertile menstrual cycle.

CFG-score D: Fig 6d shows an example for a temperature data curve (upper graph) and corresponding temperature amplitude (lower graph) of a female menstrual cycle with a short luteal phase, which may be due to limited active progesterone. The temperature increase lasts for 8 to 10 days. Further medical diagnostics may be required. A progesterone therapy may increase fertility in such cases if progesterone is given in the right time window. The present application provides a method to determine the right time window for a progesterone therapy thereby limiting to overall amount of hormones in the therapy.

CFG Score E: Fig 6e shows an example for a temperature data curve (upper graph) and corresponding temperature amplitude (lower graph) of a monophasic female menstrual cycle where no ovulation occurs. No temperature change is detectable and no significant variation in the temperature amplitude can be determined. The menstrual cycle is monophasic and no break in the homogeneity, a temperature increase and/or an increase in progesterone level is determined. No ovulation occurs in this cycle.

If a temperature increase and/or an increase in progesterone level is determined and the increase lasts for less than 6 to 7 days in CFG Score E this could indicate an example for a temperature data curve (upper graph) and corresponding temperature amplitude (lower graph) of a female menstrual cycle indicating an lutenized unrupted follicle syndrome (LUF-syndrome). The temperature may only increase slightly and the temperature increase may be less than in a normal cycle. The temperature amplitude (lower graph) does only show a slight decrease over these 6-7 days. The decrease is shorter and the difference in the amplitude is less compared to the normal "fertile" cycle and compared to the short luteal phase (CFG-score 3).This may be an indication of a so-called LUF-syndrome (luteinized unruptured follicle syndrome). Pregnancy may be difficult in such cases and a more detailed diagnostic and therapy may be required.

A break in homogeneity can be defined as a break in the measured temperature curve of at least 0.2° Celsius. If this break in temperature is positive, i.e. the break involves an increase in temperature and if the second cycle phase lasts for at least some days, the cycle may be considered as bi-phasic. Examples for bi-phasic cycles are the cycles shown in Figures 6b to 6c.

Monitoring the level of active progesterone may help to improve hormone therapies, in particular progesterone therapies. The time interval and the amount for progesterone prescribed to the user may be determined from the actual active progesterone level measured as indicated above. The total amount of additional progesterone can be limited and adapted to the individual needs.

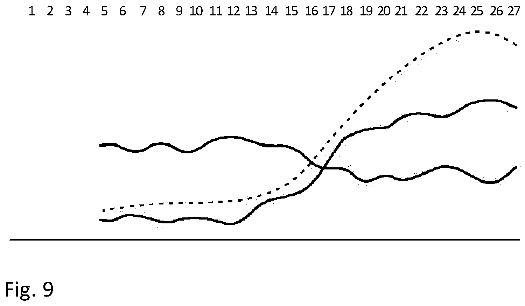

Usual progesterone measurement from blood samples indicate the level of absolute progesterone in the body. It is an indication of free progesterone available in the body. This absolute progesterone, however, is not necessarily active in the body. The inventors found that the body temperature amplitude is reduced in correlation with the amount of active progesterone, i.e. progesterone that attached to a progesterone receptor and triggered body reactions and within other the reduction in temperature amplitude. In other words, the body temperature amplitude is an indicator for a progesterone response of the body. The level of active progesterone may differ from the absolute progesterone level. For example, the absolute progesterone level is constantly increased during pregnancy, while the progesterone response and the active progesterone level is saturated. This is indicated by the constant lower temperature amplitude. Fig. 9 shows a comparison of the temperature variation, the progesterone response and the free progesterone in blood.

In some cases, the absolute progesterone level may be sufficiently high to enable and/or ensure pregnancy but the uptake of progesterone by the body may be too low resulting in an absent or low progesterone responses, indicated by a lower or no decrease in amplitude. The present disclosure may also be used to test and evaluate the functionality of the endocrinological system by monitoring the response in temperature amplitude and such the progesterone response by active progesterone to the increase in absolute progesterone. The absolute progesterone may be modified by natural processes or by medication.

A time series of temperature data points can be read out and evaluated at any time during the menstrual cycle. For example, the temperature data points may be read out or transmitted to a reader or analysis device once every 24 hours or twice a day. The temperature curve corresponds then to the present menstrual cycle of the female up to the last temperature measurement before read-out, i.e. before the pessary was removed from the vagina. The time series of temperature data points is thus in this case not a complete menstrual cycle but only a first portion. A determination of the current status can be made in real-time during the menstrual cycle.

The female cycle is determined by complex endocrinological systems. Additional parameters can help to improve determination of the status of the endocrinological systems and the female cycle. In some examples of the present disclosure, the progesterone measurement may be complimented by additional parameters. For example, absolute temperature values or other parameters such as the pH-value of the cervical mucus may be considered. The pH-information can be obtained with a measurement device and a pH-sensor can be integrated into the temperature measuring device that can be placed in the vagina. It may be sufficient for some applications to use manual pH-measurements with conventional pH-test strips at pre-determined time intervals. For example, pH measurements taken once a day may be sufficient. The measurement sequence may also be altered during the cycle and/or during therapy. Measurement of the pH-value of the cervical mucus may give additional information on pre-ovulatory processes.

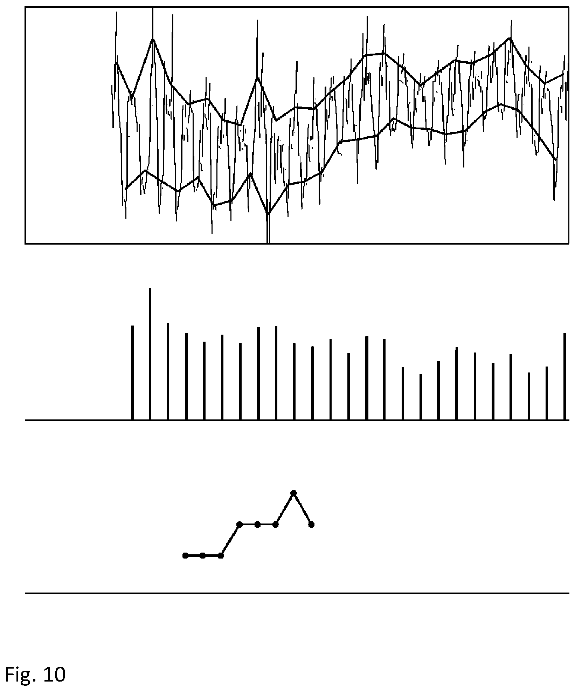

Fig. 10 shows an example of how the variation of the temperature amplitude may be correlated with pH-values of the cervical mucus. Representative maximum and minimum temperature values are determined from a set of continuous temperature data point (upper graph) and daily temperature amplitudes are determined (middle graph) as described with respect to Figure 4 and 5. Additional daily measurements of ht pH-value, for example taken with pH-strips once a day, indicate an increasing pH-value (lower graph) or more basic cervical mucus. The increase in pH correlates to a decrease in temperature amplitude (middle graph) which may indicate and/or confirm ovulation and/or progesterone delivery.

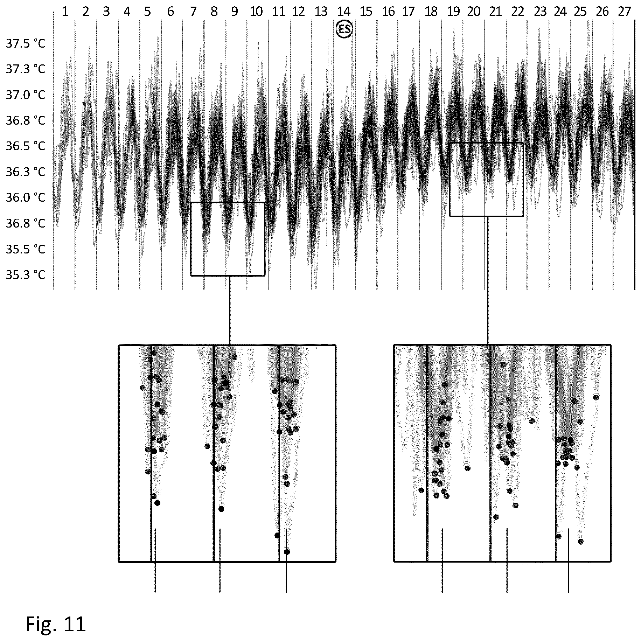

Fig. 11 relates to a further example, where nadir values are considered as additional parameter. The nadir values correspond to the lowest temperature value measured during a certain time period. The nadir value may be a representative temperature minimum during a 24 hours or daily time interval. In one aspect, the nadir value may vary over the female cycle, as for example indicated in Figure 3. The nadir value may increase by 0.3°C to 0.4°C in the luteal phase after ovulation. Experimental results shown in Table 1 show an average increase of the nadir value of 0.36°C between the follicular phase and the luteal phase. The increase in temperature can be correlated to the variation of the daily temperature amplitude. As the daily amplitude changes, the representative temperature maximums or peak values show a less significant increase depending on the progesterone level.

**Table 1**

| (median, n=11) | | changes between follicular and lutel phase |
|---|---|---|
| time of day [hZT] | nadir | 0.67 |
| | peak | 0.16 |
| | t25 | 0.35 |
| | t50 | 0.51 |
| | t75 | 0.45 |
| | | |
| position in the diurnal cycle [%] | peak | -1.32 |
| | t25 | -1.4 |
| | t50 | -0.8 |
| | t75 | -1.1 |
| | | |
| temperature [°C] | nadir | 0.36 |
| | reak | 0.26 |
| temperature [Δ°C] | amplitude | 0.14 |

The inventors further found, that the nadir values also shift in the point in time during the day when they occur.

In this respect, an illustrative method useful for understanding the invention is disclosed, the method being a method for determining a fertility status a female, the method comprising: determining, for a plurality of first time intervals, a continuous series of temperature data points relating to a body temperature; determining, in the series of data points, for each one of one of the plurality of first time intervals, a temperature minimum time; and determining a variation in the temperature minimum time, wherein the variation in the temperature minimum time indicates a change in the fertility status of the female.

The temperature minimum time is the point in time when representative temperature minimum or nadir value occurs for each first time interval, respectively. The first time interval typically is a day or a 24 hours interval the temperature minimum time may be the point time when representative temperature minimum or nadir value occurs during the day. In this respect, temperature minimum time may be termed daily temperature minimum time or nadir time. A variation or shift in the time is evaluated.

The absolute minimum temperature or nadir value is not necessarily determined but may be used as an additional or complementary parameter. For example, the minimum temperature value may vary at a different time or at a different rate compared to the shift or variation of the temperature minimum time. The point in time during the day when the representative minimum temperature or nadir is found is termed nadir time.

The inventors identified a shift in nadir time in biphasic cycles, while the nadir times stay within a time window within the follicular phase. Figure 11 shows a plurality of temperature curves of biphasic cycles plotted on top of each other. In this example, 20 temperature curves with biphasic cycles were used for simplicity but many more curves may be used to obtaining sufficient statistics. A significant variation in the nadir time is seen between the follicular phase and the luteal phase. The shift in the nadir time may be at least 0.5 hrs. The mean time shift in the nadir time was 0.67 hrs in the example shown in Fig 11 and in Table 1. This time shift is observed in biphasic cycles and can be used as an indication for a biphasic cycle in which ovulation occurred. This shift in the nadir time can be correlated to the change in the daily temperature amplitude and can be used as complementary or additional information to the progesterone level.

Temperature measurements and the evaluation methods and systems may be used and applied as explained above with respect to the variation in temperature amplitude with the difference that but the nadir time is evaluated instead of the temperature amplitude. The temperature amplitude variation may be correlated or otherwise combined with information deducted from the variation in nadir time.

The above disclosure has been given with respect to the determination of a biomarker, the hormone progesterone and the fertility status of a human female. The method and the system, however, are equally applicable with any mammalian female where a continuous temperature measurement is possible. If not related to fertility or pregnancy, the present disclosure and in particular the measurement of temperature amplitude may also be correlated to other body parameters in men and women or mammals.

## Claims

1. A method for determining a status of a biomarker in a mammal or human being, the method comprising:
- determining, for a plurality of first time intervals, a continuous series of temperature data points relating to a body temperature;
- determining, in the series of temperature data points, for the plurality of first time intervals, differences between a temperature maximum and a temperature minimum to obtain a plurality of temperature amplitudes;
- determining a series of representative temperature values for the plurality of first time intervals,
and subtracting the series of representative temperature values from the continuous series of temperature data points relating to the body temperature to obtain a series of temperature fluctuations;
wherein
- the determining, for the plurality of first time intervals, the differences between a temperature maximum and a temperature minimum to obtain a plurality of temperature amplitudes is determined from the series of temperature fluctuations;
- determining a series of temperature amplitudes from the plurality of temperature amplitudes;
- determining if a first portion of the series of temperature amplitudes varies from a second portion of the series of temperature amplitudes indicating a change in the biomarker status.

2. The method of claim 1, wherein the first portion of the series of temperature amplitudes varies from a second portion of the series of temperature amplitude in a decreased amplitude indicating a higher biomarker level.

3. The method of claim any one of the preceding claims, wherein the biomarker is a-progesterone response of the human being or mammal.

4. The method of any one of the preceding claims, wherein the series of representative temperature value is at least one of a series of Nadir values and a series of average temperature values.

5. The method of any one of the preceding claims, further comprising determining a change rate of the temperature amplitude.

6. The method of any one of the preceding claims, wherein the variation between the first portion of the series of temperature amplitudes and the second portion of the series of temperature amplitude is at least one of a variation in the average amplitude of the temperature amplitudes, a variation in the change rate of the temperature amplitudes, an increase in the temperature amplitudes or a decrease in the temperature amplitudes.

7. The method of any one of the preceding claims wherein the first portion of the series of temperature amplitudes varies from a second portion of the series of temperature amplitude in a decreased amplitude indicating a higher level of progesterone as biomarker.

8. The method of claim 7, further comprising determining the length of the second portion and characterizing the female cycle based on the length of the second portion.

9. The method of any one of claim 7 or 8, wherein the first portion of the series of temperature amplitudes varies from a second portion of the series of temperature amplitude in a decreased amplitude and wherein the second period lasts for at least 17 days, indicating a beginning pregnancy.

10. The method of any one of the preceding claims, further determining the duration of a significant change by determining a number of first time intervals, for which the representative temperature value changes.

11. The method of any one of the preceding claims, further comprising determining a series of pH-values and identifying a variation in the pH-values and correlating the variation in the pH-values with the variation between the first portion of the series of temperature amplitude and the second portion of the series of temperature amplitude.

12. The method of any one of the preceding claims, further comprising determining a representative temperature value for each one of the first time intervals and identifying a significant change in the representative temperature value and correlating the significant change to the first portion of the series of temperature amplitude and the second portion of the series of temperature amplitude.

13. A system for determining a status of a biomarker in a human being or mammal, the system comprising:
a temperature measurement device (2) for determining and recording a series of temperature data points relating to a body temperature of the female;
an analysing tool for analysing the series of temperature data points, wherein the series of temperature data points is transferred from the temperature measurement device to the analysing tool,
wherein the analysing tool performs the steps of any one of claim 1 to 12.

## Patentansprüche

1. Verfahren zum Bestimmen eines Status eines Biomarkers in einem Säugetier oder Menschen, wobei das Verfahren umfasst:
- Bestimmen, für eine Vielzahl von ersten Zeitintervallen, einer kontinuierlichen Reihe von Temperaturdatenpunkten, die sich auf eine Körpertemperatur beziehen;
- Bestimmen, in der Reihe von Temperaturdatenpunkten, für die Vielzahl von ersten Zeitintervallen, von Differenzen zwischen einem Temperaturmaximum und einem Temperaturminimum, um eine Vielzahl von Temperaturamplituden zu erhalten;
- Bestimmen einer Reihe von repräsentativen Temperaturwerten für die Vielzahl von ersten Zeitintervallen,
und Subtrahieren der Reihe von repräsentativen Temperaturwerten von der kontinuierlichen Reihe von Temperaturdatenpunkten, die sich auf die Körpertemperatur beziehen, um eine Reihe von Temperaturschwankungen zu erhalten;
wobei
- das Bestimmen, für die Vielzahl von ersten Zeitintervallen, der Differenzen zwischen einem Temperaturmaximum und einem Temperaturminimum, um eine Vielzahl von Temperaturamplituden zu erhalten, aus der Reihe von Temperaturschwankungen bestimmt wird;
- Bestimmen einer Reihe von Temperaturamplituden aus der Vielzahl von Temperaturamplituden;
- Bestimmen, ob ein erster Abschnitt der Reihe von Temperaturamplituden von einem zweiten Abschnitt der Reihe von Temperaturamplituden abweicht als Anzeichen für eine Änderung des Biomarkerstatus.

2. Verfahren gemäß Anspruch 1, wobei der erste Abschnitt der Reihe von Temperaturamplituden von einem zweiten Abschnitt der Reihe von Temperaturamplituden in einer verringerten Amplitude als Anzeichen für einen höheren Biomarkerpegel abweicht.

3. Verfahren gemäß einem der vorhergehenden Ansprüche, wobei der Biomarker eine Progesteronantwort des Menschen oder Säugetiers ist.

4. Verfahren gemäß einem der vorhergehenden Ansprüche, wobei die Reihe von repräsentativen Temperaturwerten mindestens eine aus einer Reihe von Nadirwerten und einer Reihe von Durchschnittstemperaturwerten ist.

5. Verfahren gemäß einem der vorhergehenden Ansprüche, zudem umfassend ein Bestimmen einer Änderungsrate der Temperaturamplitude.

6. Verfahren gemäß einem der vorhergehenden Ansprüche, wobei die Variation zwischen dem ersten Abschnitt der Reihe von Temperaturamplituden und dem zweiten Abschnitt der Reihe von Temperaturamplituden mindestens eine aus einer Variation der Durchschnittsamplitude der Temperaturamplituden, einer Variation der Änderungsrate der Temperaturamplituden, einer Zunahme der Temperaturamplituden oder einer Abnahme der Temperaturamplituden ist.

7. Verfahren gemäß einem der vorhergehenden Ansprüche, wobei der erste Abschnitt der Reihe von Temperaturamplituden von einem zweiten Abschnitt der Reihe von Temperaturamplituden in einer verringerten Amplitude als Anzeichen für einen höheren Progesteronpegel als Biomarker abweicht.

8. Verfahren gemäß Anspruch 7, zudem umfassend ein Bestimmen der Länge des zweiten Abschnitts und ein Charakterisieren des weiblichen Zyklus basierend auf der Länge des zweiten Abschnitts.

9. Verfahren gemäß einem der Ansprüche 7 oder 8, wobei der erste Abschnitt der Reihe von Temperaturamplituden von einem zweiten Abschnitt der Reihe von Temperaturamplituden in einer verringerten Amplitude abweicht und wobei der zweite Zeitraum mindestens 17 Tage dauert als Anzeichen für eine beginnende Schwangerschaft.

10. Verfahren gemäß einem der vorhergehenden Ansprüche, zudem umfassend ein Bestimmen der Dauer einer signifikanten Änderung durch Bestimmen einer Anzahl von ersten Zeitintervallen, für die sich der repräsentative Temperaturwert ändert.

11. Verfahren gemäß einem der vorhergehenden Ansprüche, zudem umfassend ein Bestimmen einer Reihe von pH-Werten und ein Identifizieren einer Variation der pH-Werte und ein Korrelieren der Variation der pH-Werte mit der Variation zwischen dem ersten Abschnitt der Reihe von Temperaturamplituden und dem zweiten Abschnitt der Reihe von Temperaturamplituden.

12. Verfahren gemäß einem der vorhergehenden Ansprüche, zudem umfassend ein Bestimmen eines repräsentativen Temperaturwerts für jedes der ersten Zeitintervalle und ein Identifizieren einer signifikanten Änderung des repräsentativen Temperaturwerts und ein Korrelieren der signifikanten Änderung mit dem ersten Abschnitt der Reihe von Temperaturamplituden und dem zweiten Abschnitt der Reihe von Temperaturamplituden.

13. System zum Bestimmen eines Status eines Biomarkers in einem Menschen oder Säugetier, wobei das System umfasst:
eine Temperaturmessvorrichtung (2) zum Bestimmen und Aufzeichnen einer Reihe von Temperaturdatenpunkten, die sich auf eine Körpertemperatur einer Frau beziehen;
ein Analysewerkzeug zum Analysieren der Reihe von Temperaturdatenpunkten, wobei die Reihe von Temperaturdatenpunkten von der Temperaturmessvorrichtung an das Analysewerkzeug übertragen wird,
wobei das Analysewerkzeug die Schritte gemäß einem der Ansprüche 1 bis 12 durchführt.

## Revendications

1. Procédé de détermination d'un état d'un biomarqueur chez un mammifère ou un être humain, le procédé comprenant :
- la détermination, pour une pluralité de premiers intervalles de temps, d'une série continue de points de données de température relatifs à une température corporelle ;
- la détermination, dans la série de points de données de température, pour la pluralité de premiers intervalles de temps, de différences entre un maximum de température et un minimum de température pour obtenir une pluralité d'amplitudes de température ;
- la détermination d'une série de valeurs de température représentatives pour la pluralité de premiers intervalles de temps,
et la soustraction de la série de valeurs de température représentatives de la série continue de points de données de température relatifs à la température corporelle pour obtenir une série de fluctuations de température ;
dans lequel
- la détermination, pour la pluralité de premiers intervalles de temps, les différences entre un maximum de température et un minimum de température pour obtenir une pluralité d'amplitudes de température est déterminée à partir de la série de fluctuations de température ;
- la détermination d'une série d'amplitudes de température à partir de la pluralité d'amplitudes de température ;
- la détermination si une première partie de la série d'amplitudes de température varie par rapport à une seconde partie de la série d'amplitudes de température indiquant un changement dans l'état du biomarqueur.

2. Procédé selon la revendication 1, dans lequel la première partie de la série d'amplitudes de température varie par rapport à une seconde partie de la série d'amplitudes de température dans une amplitude diminuée indiquant un niveau de biomarqueur plus élevé.

3. Procédé selon l'une quelconque des revendications précédentes, dans lequel le biomarqueur est une réponse à la progestérone de l'être humain ou du mammifère.

4. Procédé selon l'une quelconque des revendications précédentes, dans lequel la série de valeurs de température représentatives est au moins l'une d'une série de valeurs de nadir et d'une série de valeurs de température moyennes.

5. Procédé selon l'une quelconque des revendications précédentes, comprenant en outre la détermination d'un gradient de changement de l'amplitude de température.

6. Procédé selon l'une quelconque des revendications précédentes, dans lequel la variation entre la première partie de la série d'amplitudes de température et la seconde partie de la série d'amplitudes de température est au moins l'une d'une variation de l'amplitude moyenne des amplitudes de température, d'une variation du gradient de changement des amplitudes de température, d'une augmentation des amplitudes de température ou d'une diminution des amplitudes de température.

7. Procédé selon l'une quelconque des revendications précédentes, dans lequel la première partie de la série d'amplitudes de température varie par rapport à une seconde partie de la série d'amplitudes de température dans une amplitude diminuée indiquant un niveau plus élevé de progestérone en tant que biomarqueur.

8. Procédé selon la revendication 7, comprenant en outre la détermination de la longueur de la seconde partie et la caractérisation du cycle féminin sur la base de la longueur de la seconde partie.

9. Procédé selon l'une quelconque des revendications 7 ou 8, dans lequel la première partie de la série d'amplitudes de température varie par rapport à une seconde partie de la série d'amplitudes de température dans une amplitude diminuée et dans lequel la seconde période dure au moins 17 jours, indiquant un début de grossesse.

10. Procédé selon l'une quelconque des revendications précédentes, déterminant en outre la durée d'un changement significatif en déterminant un nombre de premiers intervalles de temps, pour lesquels la valeur de température représentative change.

11. Procédé selon l'une quelconque des revendications précédentes, comprenant en outre la détermination d'une série de valeurs de pH et l'identification d'une variation des valeurs de pH et la corrélation de la variation des valeurs de pH avec la variation entre la première partie de la série d'amplitudes de température et la seconde partie de la série d'amplitudes de température.

12. Procédé selon l'une quelconque des revendications précédentes, comprenant en outre la détermination d'une valeur de température représentative pour chacun des premiers intervalles de temps et l'identification d'un changement significatif de la valeur de température représentative et la corrélation du changement significatif avec la première partie de la série d'amplitudes de température et la seconde partie de la série d'amplitudes de température.

13. Système de détermination d'un état d'un biomarqueur chez un être humain ou un mammifère, le système comprenant :
un dispositif de mesure de température (2) pour déterminer et enregistrer une série de points de données de température relatifs à une température corporelle de la femme ;
un outil d'analyse pour analyser la série de points de données de température, dans lequel la série de points de données de température est transférée du dispositif de mesure de température à l'outil d'analyse,
dans lequel l'outil d'analyse effectue les étapes de l'une quelconque des revendications 1 à 12.
